# EUROPEAN PATENT APPLICATION

(11) **EP 3 459 508 A1**
(43) Date of publication of application: **27.03.2019**
(21) Application number: 17192485.5
(22) Date of filing: 21.09.2017
(51) Int. Cl.: A61F 7/00, A61F 7/02

(54) **FORCED AIR WARMING BLANKET**

(71) Applicant: The Surgical Company International B.V., 3821 AH Amersfoort (NL)
(72) Inventor: TEUNISSEN, Berend Jan, NL-7481JN Haaksbergen (NL)
(74) Representative: Hindles Limited

(57) **Abstract**

The invention relates to a forced air warming blanket for regulating a temperature of a patient, the blanket having a patient drape detachably connected thereto.

## Description

### Field of the invention

The invention relates to forced air warming blankets, methods of manufacturing forced air warming blankets and methods of using forced air warming blankets.

### Background to the invention

Forced air warming blankets are used over the bodies of human patients to regulate their temperatures during surgery to thereby protect them against unintended hypothermia. Patient drapes (e.g. face masks/shields) are used with forced air warming blankets to cover the head of the patient to inhibit heat loss and to shield the face of the patient from blood spatter. Such drapes are also used on neonates to cover part of the body. The drapes are transparent for observing the face and skin of the patient.

For convenience, the drape may be pre-attached to an appropriate region of the forced air warming blanket. One way of automatically manufacturing forced air warming blankets involves continuously translating one or more constituent layers of the blankets in a principal manufacturing direction (typically in an assembly line arrangement). However, it is difficult to adhere a patient drape to the blanket in a direction perpendicular to the manufacturing direction during such an automated manufacturing process because the continuous motion of the blanket in the manufacturing direction makes it difficult to deposit a line of adhesive (with which to attach the drape) in a direction perpendicular to the manufacturing direction. Accordingly, particularly where drapes need to be attached to the forced air warming blanket along a line perpendicular to the manufacturing direction, they are not pre-attached to the forced air warming blanket. Instead, a separate patient drape is provided which is then attached to an appropriate region of the forced air warming blanket in use so that it can perform the above functions. However, this can be inconvenient for practitioners.

Accordingly, improved forced air warming blankets, ways of manufacturing forced air warming blankets and ways of using forced air warming blankets are required.

### Summary of the invention

A first aspect of the invention provides a forced air warming blanket for regulating a temperature of a (typically human) patient (typically during a medical or surgical procedure or operation), the blanket having a patient drape (typically for covering a patient's face in use) detachably connected thereto.

By detachably connecting the patient drape to the forced air warming blanket, the forced air warming blanket can be automatically manufactured with the drape connected thereto in a position and/or orientation other than its optimal use position and/or orientation, but later detached from the forced air warming blanket and relocated and/or re-oriented (typically relative to the forced air warming blanket) to its optimal use position and/or orientation in use. This allows the manufacturing process to be optimised independently of the optimum use position and/or orientation of the drape, whilst still allowing the drape to be connected to the forced air warming blanket during manufacture (which remains convenient for the medical practitioner ultimately using the blanket to regulate the temperature of a patient).

Typically the patient drape is detachably connected to the blanket such that the drape is fully detachable from the blanket, typically such that no physical connection remains between the drape and the blanket when the drape is detached from the blanket.

It may be that the forced air warming blanket is disposable.

It may be that the patient drape is disposable.

It may be that the forced air warming blanket is configured to regulate the temperature of a patient by forced convection. It may be that the forced air warming blanket comprises an air chamber having an air impermeable layer on a first side facing away from the patient in use and an air permeable layer on a second side opposite the first side and facing the patient in use. It may be that the air impermeable layer (and/or the air permeable layer) has a port extending therethrough and configured to receive heated, pressurised air from a forced air warmer. It may be that the air permeable layer allows the passage of air therethrough onto the patient (e.g. by way of openings or perforations in the said air permeable layer).

It may be that the blanket is configured to cover at least part of a front side of a patient.

It may be that the blanket is configured to cover at least part of a back side of a patient.

It may be that the patient drape is transparent for (a human) observing a portion of a patient beneath the drape in use.

It may be that the patient drape comprises or consists of a face mask or face shield.

It may be that the patient drape is for use with the forced air warming blanket to thereby regulate the temperature of the patient.

It may be that the patient drape is configured to inhibit (or prevent) the passage of blood therethrough. It may be that the patient drape is impervious to blood.

It may be that the patient drape is configured to inhibit passage of (heated) air therethrough. It may be that the patient drape is impervious to air.

It may be that the patient drape is formed from a plastic material.

Typically the patient drape is attached to the forced air warming blanket by way of a blanket connection adhesive.

Typically the forced air warming blanket is elongate along a longitudinal axis.

Typically the blanket connection adhesive is arranged along a line extending at an angle of less than 45° to a direction parallel to the longitudinal axis of the forced air warming blanket (or to a direction parallel to longitudinal axes of one or more constituent layers of the forced air warming blanket).

Typically the forced air warming blanket is manufactured by way of an assembly line which involves translating one or more constituent layers of the forced air warming blanket in a principal manufacturing direction. The principal manufacturing direction is typically parallel or substantially parallel to the longitudinal axis of the blanket (or parallel to longitudinal axes of one or more constituent layers of the forced air warming blanket, typically including the layer on which the drape is adhered). The manufacturing process can typically be optimised by depositing the blanket connection adhesive on a (typically top) surface of the forced air warming blanket along a line parallel or substantially parallel to the said manufacturing direction. Accordingly, it may be that the said blanket connection adhesive is arranged along a line parallel or substantially parallel to the said longitudinal axis (or axes).

By depositing blanket connection adhesive along a line substantially parallel to the manufacturing direction, we typically mean depositing blanket connection adhesive along a line which deviates (typically in a plane of the said surface on which the blanket connection adhesive is provided) from a direction parallel to the manufacturing direction by less than 10°, preferably less than 5°.

By the blanket connection adhesive being arranged along a line substantially parallel to the longitudinal axis of the blanket (or longitudinal axes of the said layers), we typically mean the blanket connection adhesive being arranged along a line which deviates (e.g. in a plane of a surface of the said blanket on which the blanket connection adhesive is provided) from a direction parallel to the longitudinal axis of the blanket (or longitudinal axes of said layers) by less than 10°, preferably less than 5°.

It may be that the patient drape comprises a drape relocation adhesive provided thereon, typically covered by a release liner. Typically the said release liner is removable to thereby allow the drape (e.g. after it has been detached from the blanket) to be attached (or re-attached) to the blanket or to a patient or to a garment worn by a patient by way of the drape relocation adhesive. Alternatively, it may be that the patient drape is used (e.g. after it has been detached from the blanket) without attaching (or re-attaching) it to the blanket or the patient or to a garment worn by the patient (e.g. it may simply be draped over the patient).

The drape relocation adhesive is typically spaced from the blanket connection adhesive. Nevertheless, the drape relocation adhesive may be (but is not necessarily) the same type of adhesive as the blanket connection adhesive (indeed they may be identical formulations).

It may be that the drape relocation adhesive is arranged along a line parallel or substantially parallel to the longitudinal axis of the blanket (or the longitudinal axes of one or more constituent layers of the said blanket, typically including the layer on which the drape is adhered). It may be that the drape relocation adhesive is arranged along a line parallel or substantially parallel to the line in which the blanket connection adhesive is arranged.

By drape relocation adhesive being arranged along a line substantially parallel to the longitudinal axis of the blanket (or to longitudinal axes of said one or more layers), we typically mean the drape relocation adhesive is arranged along a line which deviates (typically in a plane of a surface of the said drape on which the drape relocation adhesive is provided) from a direction parallel to the said longitudinal axis (or axes) by less than 10°, preferably less than 5°.

By drape relocation adhesive being arranged along a line substantially parallel to the line in which the blanket connection adhesive is arranged, we typically mean the drape relocation adhesive is arranged along a line which deviates (typically in a plane of a surface of the said drape on which the drape relocation adhesive is provided) from a direction parallel to the said the line in which the blanket connection adhesive is arranged by less than 10°, preferably less than 5°.

It may be that the blanket connection adhesive comprises a thermoplastic (e.g. hotmelt) adhesive.

It may be that the drape relocation adhesive comprises a polymer adhesive layer.

It may be that the forced air warming blanket comprises one or more visible (i.e. visible to humans) markings identifying the position of the patient drape on the forced air warming blanket.

Typically the patient drape is detachable from the forced air warming blanket without substantively affecting or impairing the function of the forced air warming blanket.

Typically the patient drape is detachable from the forced air warming blanket by hand. That is, the patient drape is typically readily detachable from the forced air warming blanket by a human using their hands but without additional tools.

Typically the patient drape is detachably connected the forced air warming blanket by way of a breakable (e.g. tearable, e.g. by a human by hand) connection (e.g. a breakable (e.g. tearable) strip). Typically the breakable connection is breakable to thereby release the patient drape from the forced air warming blanket.

It may be that the said breakable connection is provided (axially) between the patient drape and a joint connecting the patient drape to the forced air warming blanket comprising the blanket connection adhesive.

It may be that the said breakable connection comprises one or more perforations (typically a plurality of perforations). It may be that the said breakable connection comprises a plurality of perforations arranged along a line. Typically the said line is parallel or substantially parallel to the line in which the blanket connection adhesive is arranged. Typically the said line is parallel or substantially parallel to the longitudinal axis of the forced air warming blanket (or longitudinal axes of one or more constituent layers of the forced air warming blanket).

By the perforation line being substantially parallel to the line in which the blanket connection adhesive is arranged, we typically mean that the said perforation line deviates (typically in a plane of a surface of the drape) from the direction in which the line of blanket connection adhesive is arranged by less than 10°, preferably less than 5°.

By the perforation line being substantially parallel to the said longitudinal axis (or axes), we typically mean that the said perforation line deviates (typically in a plane of a surface of the drape) from a direction parallel to said axis (or axes) by less than 10°, preferably less than 5°.

A second aspect of the invention provides a (typically automated) method of manufacturing a forced air warming blanket for regulating a temperature of a (typically human) patient (typically during a medical or surgical procedure or operation), the method comprising: (typically continuously) translating one or more constituent layers of the forced air warming blanket along a principal manufacturing direction; depositing a blanket connection adhesive on a (typically top or uppermost) said constituent layer of the forced air warming blanket along a line parallel or substantially parallel to the principal manufacturing direction; and attaching a detachable patient drape to the forced air warming blanket by way of the blanket connection adhesive.

Typically the patient drape attached to the blanket is fully detachable from the blanket, typically such that no physical connection remains between the drape and the blanket after the drape has been detached from the blanket.

By depositing the blanket connection adhesive along a line parallel or substantially parallel to the principal manufacturing direction and attaching the patient drape to the forced air warming blanket by way of the blanket connection adhesive, the forced air warming blanket can be manufactured more easily than if, for example, the blanket connection adhesive had to be deposited in a direction perpendicular to the principal manufacturing direction. In addition, by making the patient drape detachable, it can be detached from the forced air warming blanket and relocated and/or re-oriented (typically relative to the forced air warming blanket) in use. Accordingly, the second aspect of the invention allows the patient drape to be attached to the forced air warming blanket at a position and/or orientation which makes manufacturing easier (which remains convenient for the medical practitioner ultimately using the blanket to regulate the temperature of a patient), but later detached from the forced air warming blanket and relocated and/or re-oriented (typically relative to the forced air warming blanket) to its optimal use position and/or orientation in use.

By a line substantially parallel to the principal manufacturing direction, we typically mean a line which deviates from a direction parallel to the principal manufacturing direction by less than 10° or less than 5°.

Typically the patient drape is detachable from the forced air warming blanket, typically by way of a breakable connection (typically breakable by hand).

A third aspect of the invention provides a forced air warming blanket manufactured by the method of the second aspect of the invention.

A fourth aspect of the invention provides a method of using a forced air warming blanket for regulating a temperature of a (typically human) patient (typically during a medical or surgical procedure or operation), the forced air warming blanket having a patient drape detachably connected thereto, the method comprising: detaching the patient drape from the forced air warming blanket; and relocating and/or re-orienting the patient drape.

Typically the step of detaching the patient drape from the forced air warming blanket comprises fully detaching the patient drape from the blanket, typically such that no physical connection remains between the drape and the blanket after the drape has been detached from the blanket.

By (typically fully) detaching the patient drape from the forced air warming blanket and (typically subsequently) relocating and/or re-orienting the patient drape in use, the patient drape can be detachably connected to the forced air warming blanket during manufacture at a position and/or orientation most suited to the manufacturing process before being later detached and relocated and/or re-oriented to a more optimal position and/or orientation in use.

The step of re-orienting the drape may comprise rotating the drape by 90° (or by an angle between 45° and 90°, or by an angle between 80° and 90°). For example, the step of re-orienting the drape may comprise rotating the drape by 90° (or by an angle between 45° and 90°, or by an angle between 80° and 90°) in the plane of a (typically planar) surface of the blanket (e.g. a surface of the blanket to which the drape is attached before or after detaching the drape from the blanket) or a plane parallel thereto, or in the plane of a (typically planar) surface of the drape or a plane parallel thereto.

Typically the method further comprises regulating a temperature of a patient by way of the forced air warming blanket (and typically the patient drape) after the steps of detaching and relocating and/or re-orienting the patient drape. It may be that the step of regulating the temperature of the patient comprises the patient drape inhibiting heat loss, e.g. from between a gap between the forced air warming blanket and the patient (e.g. the head of the patient). It may be that the method comprises the patient drape shielding the patient (e.g. the head of the patient) from blood spatter.

It may be that the method comprises relocating and/or re-orienting the patient drape to thereby cover a portion of a patient. It may be that the method comprises relocating and/or re-orienting the patient drape to thereby inhibit blood spattering on the patient. It may be that the method comprises relocating and/or re-orienting the patient drape to thereby direct air heated by the forced air warming blanket towards the patient.

It may be that the step of relocating and/or re-orienting the patient drape comprises adhering the relocated and/or re-oriented patient drape to the blanket or to a or the patient or to a garment worn by the patient (e.g. by way of a drape relocation adhesive).

Typically the method further comprises regulating a temperature of a patient by way of the forced air warming blanket (and typically the patient drape) after the step of adhering the relocated and/or re-oriented patient drape to the blanket or to a or the patient or to a garment worn by the patient.

Typically, the drape is relocated and/or re-oriented such that the relocation adhesive is arranged along a line perpendicular or substantially perpendicular to the longitudinal axis of the blanket (or longitudinal axes of one or more constituent layers of the blanket).

By the relocation adhesive being arranged along a line substantially perpendicular to the longitudinal axis of the blanket (or longitudinal axes of one or more constituent layers of the blanket), we typically mean the relocation adhesive is arranged along a line which deviates from a direction perpendicular to the longitudinal axis of the blanket by less than 10°, preferably less than 5°.

It may be that the step of adhering the patient drape to the blanket or to a or the patient comprises removing a release liner from a drape relocation adhesive provided on the drape and adhering the patient drape to the blanket or to the patient by way of the drape relocation adhesive.

It may be that the method comprises re-orienting the drape relocation adhesive relative to the forced air warming blanket (e.g. in a plane of a (typically planar) surface of the blanket, such as a surface to which the drape is attached before or after it is detached from the blanket, or a plane parallel thereto, or in the plane of a (typically planar) surface of the drape (such as the surface on which the relocation adhesive is provided) or a plane parallel thereto) between the steps of detaching the patient drape from the forced air warming blanket and adhering the patient drape to the blanket or to the patient by way of the drape relocation adhesive.

It may be that the step of relocating and/or re-orienting the patient drape comprises re-orienting the patient drape relative to the forced air warming blanket (e.g. in a plane of a surface of the permeable or impermeable layer of the blanket, such as a surface to which the drape is attached before or after it is detached from the blanket, or a plane parallel thereto).

It may be that the step of detaching the patient drape from the forced air warming blanket comprises detaching the patient drape from the forced air warming blanket by hand.

Although various aspects and embodiments of the present invention have been described separately above, any of the aspects and features of the present invention can be used in conjunction with any other aspect, embodiment or feature where appropriate. For example apparatus features may where appropriate be interchanged with method features.

### Description of the Drawings

An example embodiment of the present invention will now be illustrated with reference to the following Figures in which:
Figures 1a and 1b are perspective views of (deflated) forced air warming blankets having patient drapes attached thereto;
Figure 2a is a schematic diagram of a forced air warming blanket having a patient drape detachably connected thereto;
Figure 2b is a flow chart illustrating a method of manufacturing a forced air warming blanket;
Figure 2c shows the forced air warming blanket of Figure 2a during manufacture before the patient drape is attached;
Figure 3 is a close-up view of the patient drape and its detachable connection to the forced air warming blanket;
Figure 4 shows schematically the diagram of Figure 2 with the patient drape being relocated and re-oriented;
Figure 5 shows the diagram of Figure 4 with the patient drape adhered to the forced air warming blanket at its new location and orientation, the patient drape being unfolded for use;
Figure 6a shows an air permeable side of an inflated alternative forced air warming blanket;
Figure 6b shows an air impermeable side of the inflated forced air warming blanket of Figure 6a;
Figure 6c shows the inflated forced air warming blanket of Figures 6a, 6b in use on a child patient;
Figures 7a and b show respectively air impermeable and air permeable sides of another alternative (inflated) forced air warming blanket;
Figures 7c and 7d show respectively air impermeable and air permeable sides of the blanket of Figures 7a, 7b deflated;
Figure 7e shows the forced air warming blanket of Figures 7a-7d inflated in use on the underside of a patient to help regulate the temperature of the patient;
Figures 8a, 8b show the air permeable side of another alternative forced air warming blanket inflated (Figure 8a) and deflated (Figure 8b);
Figure 8c shows the forced air warming blanket of Figures 8a-8b inflated in use on the underside of a patient to help regulate the temperature of the patient;
Figures 9a, 9b show the air permeable side of another alternative forced air warming blanket inflated (Figure 9a) and deflated (Figure 9b); and
Figure 9c shows the forced air warming blanket of Figures 9a-9b inflated in use on the underside of an upper body of a patient to help regulate the temperature of the patient.

### Detailed Description of an Example Embodiment

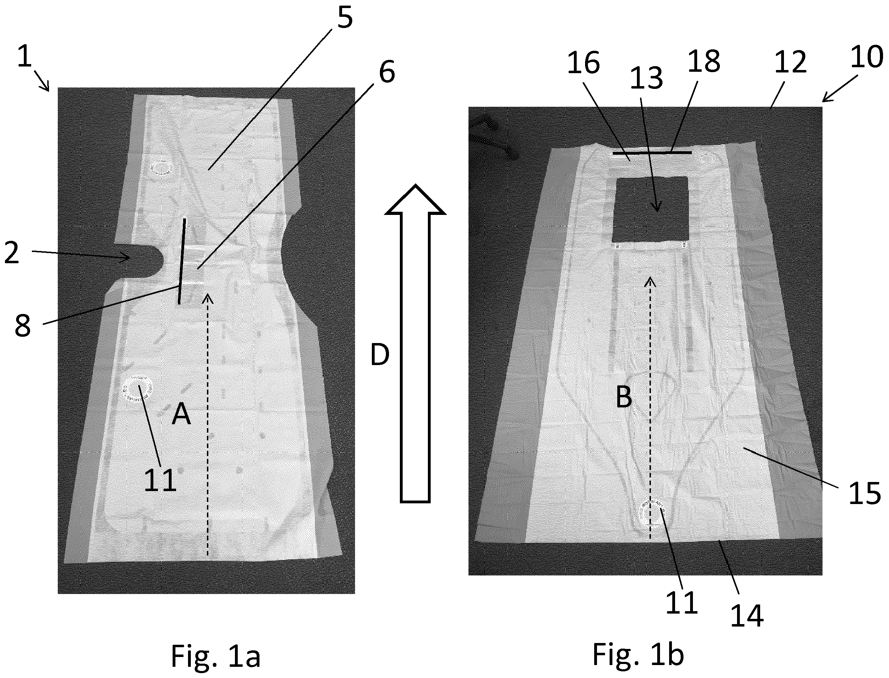

Figures 1a and 1b are perspective views of respective forced air warming blankets 1, 10 for regulating the temperature of a human patient by forced convection. The forced air warming blanket 1 of Figure 1a is elongate along a longitudinal axis A and is intended to be laid across the upper body of the patient in use such that a recess 2 of the blanket 1 receives the neck of the patient, the longitudinal axis A of the blanket 1 being oriented generally perpendicular to the length of the patient. The forced air warming blanket 10 of Figure 1b is elongate along a longitudinal axis B and is intended to be laid along the body of the patient in use such that a cut-out 13 provided at an intermediate portion of the blanket 10 along its length closer to the top end 12 than to the bottom end 14 is provided over the chest of the patient. The top end 12 of the blanket 10 is provided at the neck of the patient in use, while the bottom end 14 of the blanket 10 is provided at the feet of the patient in use.

Each of the forced air warming blankets 1, 10 comprises an internal air chamber having an air impermeable layer on a first side (the top sides 5, 15 of blankets 1, 10 in the views of Figures 1a, 1b) facing away from the patient in use and an air permeable layer on a second side (the undersides of blankets 1, 10 in the views of Figures 1 a, 1 b) opposite the first side and facing the patient in use. The air impermeable layers 5, 15 (and/or the air permeable layers) of the blankets 1, 10 have respective ports 11 extending therethrough and configured to receive heated, pressurised air from a forced air warmer through a hose. The air permeable layers allow the passage of received, heated air therethrough onto the patient in use (e.g. by way of openings or perforations in the said air permeable layers).

Each of the forced air warming blankets 1, 10 has a patient drape (typically a transparent flexible plastic face shield) 6, 16 attached thereto by way of a blanket connection adhesive. The patient drapes 6, 16 are located and oriented so that, in use, the medical practitioner can pull the drapes over the face (and typically the head) of the patient to thereby shield the face of the patient from blood spatter and to inhibit the loss of air heated by the forced air warming blankets 1, 10 from gaps between the blankets 6, 16 and the patient's head.

The forced air warming blankets 1, 10 are manufactured on an assembly line in which constituent layers of the blankets 1, 10 are translated in a manufacturing direction D (illustrated by the arrow between Figures 1a, 1b). More specifically, an air permeable layer (typically provided in the form of a sheet and typically also having previously been provided with perforations/openings) is typically laid on a conveyor belt which constantly and continuously translates the air permeable layer along the manufacturing direction D. As the air permeable layer is being translated in the manufacturing direction D, an air impermeable layer (typically also being provided in the form of a sheet and typically having previously been provided with the said port 11 therethrough) is laid over the air permeable layer. Heat and pressure are then applied to connect the air permeable and air impermeable layers together by way of a plurality of heat seal welds (typically including a peripheral weld around the perimeters of one or both the air permeable and air impermeable layers) to form the air chamber therebetween. The welds are typically arranged to connect the air permeable and air impermeable layers such that they form a panel-like shape when the finished blanket is inflated in order to evenly distribute air across the blanket (and consequently in order to evenly distribute air across the patient in use) and to prevent ballooning of the blanket in use, for example in one of the ways described in WO2010/080024 which is hereby incorporated in full herein by reference.

In respect of the blanket 1 of Figure 1a, after the air impermeable layer is deposited on the air permeable layer but before the said layers are heat sealed together, (typically thermoplastic, typically hot-melt) blanket connection adhesive is deposited along a line 8 (either by way of a continuous line of adhesive or by way of discrete portions of adhesive arranged along the line but spaced apart from each other along that line) on the air impermeable layer 5 while the constituent air permeable and air impermeable layers of the blanket 1 are being translated in the manufacturing direction D. The adhesive is typically deposited from a reservoir by a single nozzle provided above the air impermeable layer 5 and, as the constituent air permeable and air impermeable layers of the blanket 1 are being translated in the manufacturing direction D, the adhesive is deposited along line 8 parallel to the manufacturing direction D. The patient drape 6 is then laid on the blanket 1 over the blanket connection adhesive. Next, heat and pressure are applied to heat seal the air impermeable and air permeable layers together by way of the welds and to melt the blanket connection adhesive. The heat and pressure are then removed to leave the drape 6 attached to the blanket 1 by the (cooled, solidified) blanket connection adhesive.

In this case, in order to provide the drape 6 in its optimal position and orientation for use (i.e. so that the drape 6 can be pulled over the patient's head after the blanket 1 has been provided over the patient), the line 8 along which the blanket connection adhesive needs to be provided is parallel to the direction D. This allows the manufacturing process to be highly robust and repeatable. Accordingly, there is no incompatibility between the optimal use position and orientation of the drape 6 on the blanket 1 and the most robust and repeatable manufacturing process.

In respect of the blanket 10 of Figure 1b, however, in order to provide the drape 16 in its optimal position and orientation for use (i.e. so that the drape 16 can be pulled over the patient's head after the blanket 10 has been provided over the patient), the line 18 along which the blanket connection adhesive would need to be provided is perpendicular to the manufacturing direction D. Due to the constant and continuous motion of the constituent air permeable and air impermeable layers of the forced air warming blanket 10 in the direction D during manufacture, it is more difficult to robustly and repeatably deposit the blanket connection adhesive along a line 18 perpendicular to the direction D by a single nozzle as in the above mentioned manufacturing process. Accordingly, there is an incompatibility between the optimal use position and orientation of the drape 16 on the blanket 10 and the most robust and repeatable manufacturing process.

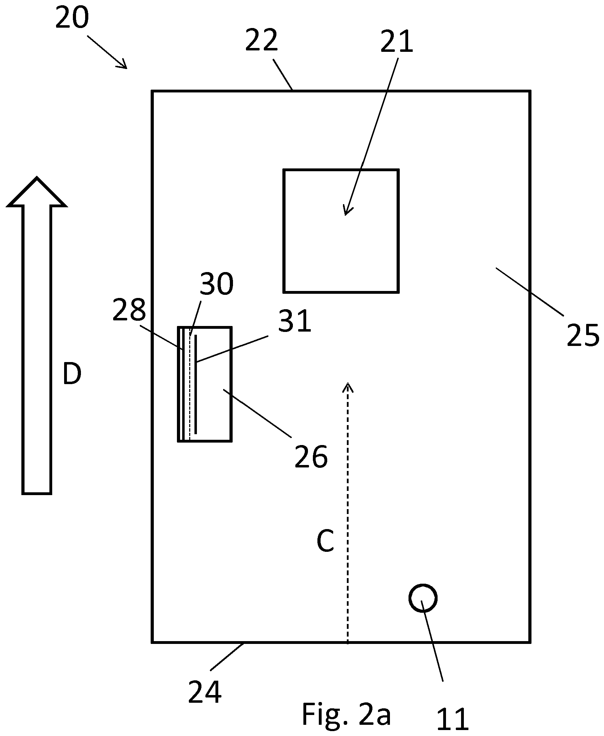

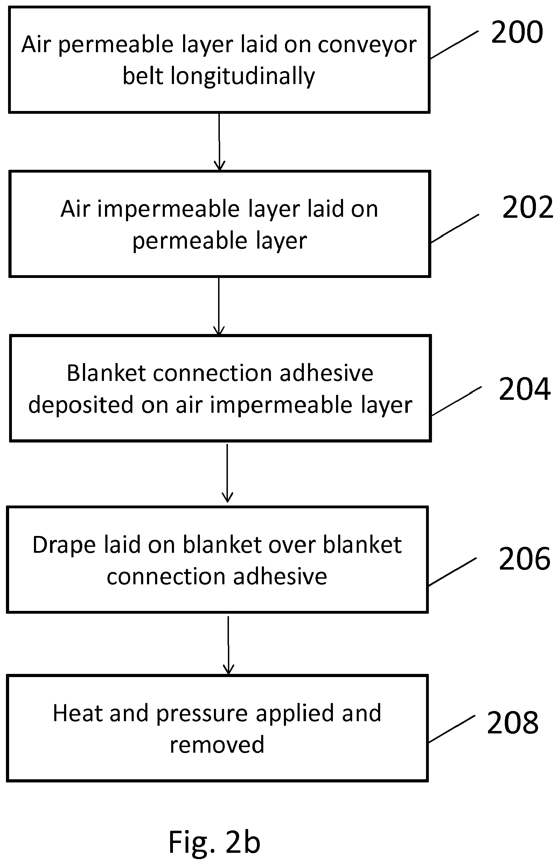

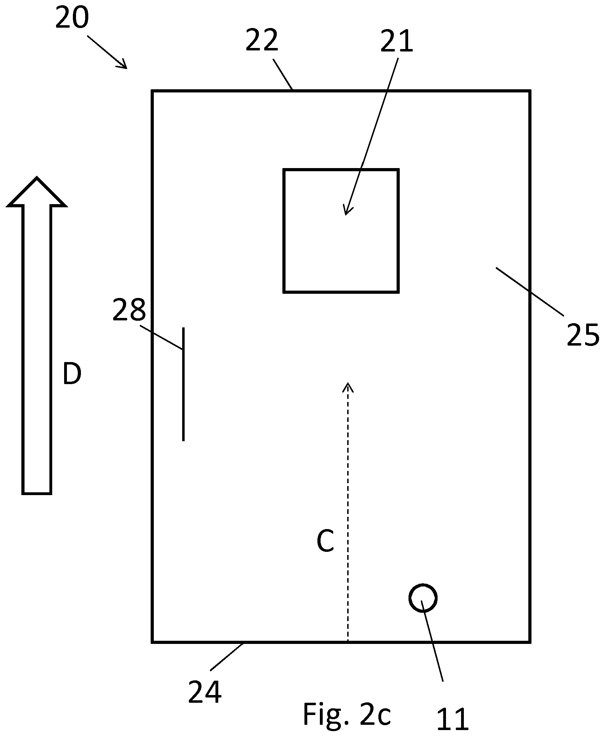

Figure 2a is a schematic plan view of an alternative forced air warming blanket 20 which is similar to the forced air warming blanket 10 of Figure 1 b. The blanket 20 is elongate along a longitudinal axis C and is intended to be laid along the body of the patient in use such that a cut-out 21 provided at an intermediate portion of the blanket 20 along its length closer to the top end 22 than to the bottom end 24 is provided over the chest of the patient. The top end 22 of the blanket 20 is provided at the neck of the patient in use, while the bottom end 24 of the blanket 20 is provided at the feet of the patient in use. The blanket 20 comprises an internal air chamber having an air permeable layer on a first side (the underside of the blanket 20 in the view of Figure 2) facing away from the patient in use and an air impermeable layer on a second side (the top side 25 of the blanket 20 in the view of Figure 2a) opposite the first side and facing the patient in use. The air impermeable layer 25 (and/or the air permeable layer) of the blanket 20 has a port 11 extending therethrough and configured to receive heated, pressurised air from a forced air warmer through a hose (not shown). The air permeable layer allows the passage of (received, heated) air therethrough onto the patient in use (e.g. by way of openings or perforations in the said air permeable layer).

The blanket 20 is manufactured using the same process as that used to manufacture the blanket 1 described above, namely (as illustrated in the flow chart of Figure 2b) in step 200 an air permeable layer (typically having previously been provided with perforations/openings) is laid on a conveyor belt which constantly and continuously translates the air permeable layer in a manufacturing direction D. As the air permeable layer is being translated in the manufacturing direction D, in step 202 an air impermeable layer 25 (typically having previously been provided with the said port 11 therethrough) is laid over the air permeable layer. In step 208 heat and pressure are then applied to connect the air permeable and air impermeable layers together by way of a plurality of heat seal welds (typically including a peripheral weld around the perimeters of one or both the air permeable and air impermeable layers) to form the air chamber therebetween. As before, the welds are typically arranged to connect the air permeable and air impermeable layers such that they form a panel-like shape when the finished blanket is inflated in order to evenly distribute air across the blanket (and consequently in order to evenly distribute air across the patient) and to prevent ballooning of the blanket in use, for example in one of the ways described in WO2010/080024 which is hereby incorporated in full herein by reference.

As shown in Figure 2c, after the air impermeable layer is deposited on the air permeable layer in step 202 but before the said layers are heat sealed together in step 208, in step 204 a (typically thermoplastic, typically hot-melt) blanket connection adhesive is deposited along a line 28 (either by way of a continuous line of adhesive or by way of discrete portions of adhesive arranged along the line but spaced apart from each other along that line) on the air impermeable layer 25 of the blanket 20 as the constituent air permeable and air impermeable layers of the blanket 20 are translated in the manufacturing direction D. The adhesive is typically deposited from a single nozzle provided above the air impermeable layer 5 and, as the constituent air permeable and air impermeable layers of the blanket 1 are being translated in the manufacturing direction D, the adhesive is deposited along a line parallel to the manufacturing direction D. In step 206 (which is between steps 204 and 208), a patient drape 26 is then laid on the blanket 1 over the blanket connection adhesive. Next, the heat and pressure are applied in step 208 as set out above to heat seal the air impermeable and air permeable layers together by way of the welds, and also to melt the blanket connection adhesive. The heat and pressure are then removed to leave the drape 26 attached to the blanket 20 by the (cooled, solidified) blanket connection adhesive (as shown in Figure 2a).

As in the embodiment of Figure 1a, the line 28 along which the blanket connection adhesive is arranged is parallel to the manufacturing direction D and to the longitudinal axis C of the blanket 20 (and parallel to longitudinal axes of the air permeable and air impermeable layers of the blanket 20). Accordingly, the drape 26 can be attached to the air permeable layer 25 robustly and repeatably by way of the automated assembly line process. However, the drape 26 is not located at its optimal location nor is it oriented in its optimal orientation for use (i.e. so that the drape 26 can be pulled over the patient's head after the blanket 20 has been provided over the patient). Rather, as set out above in respect of the forced air warming blanket 10 of Figure 1b, the optimal position of the drape 26 for use is between the cut-out 21 and the top end 22 of the blanket 20 and the drape 26 would need to be rotated 90° clockwise to be in its optimal orientation for use.

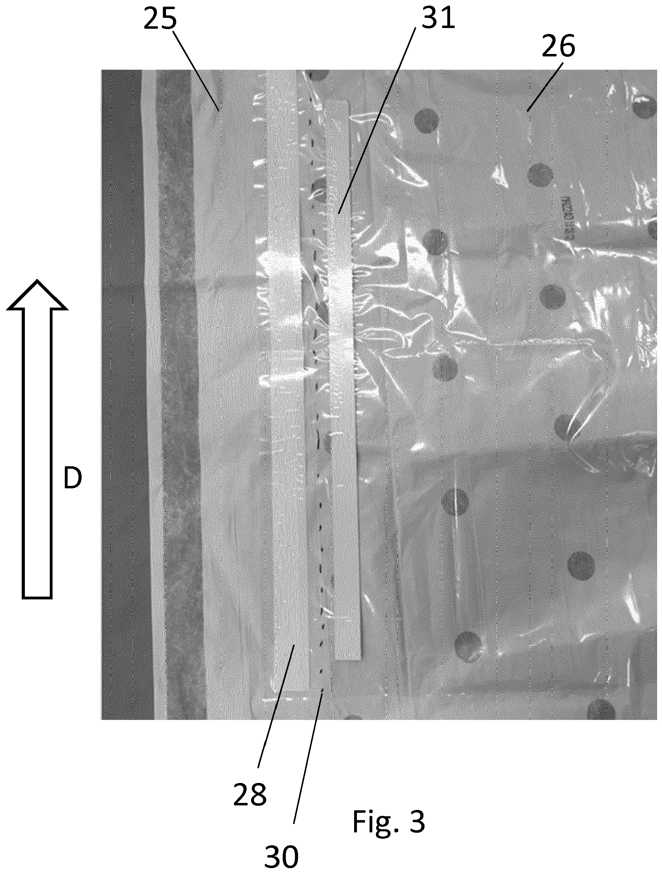

In order to overcome this issue, the drape 26 is detachably connected to the blanket 20 by way of a perforated connection 30 (see Figure 2a) provided between the drape 26 and the blanket connection adhesive 28, the perforated connection being breakable by hand (i.e. without additional tools) for convenience in use (see below). In addition, the drape 26 is provided with a relocation adhesive (which is typically a polymer adhesive layer) thereon. The relocation adhesive is arranged along a line 31 parallel to the line 28 in which the blanket adhesive is deposited and parallel to the longitudinal axis C of the blanket 20 (and parallel to longitudinal axes of the air permeable and air impermeable layers of the blanket 20). The relocation adhesive is covered by a release liner (such as a release paper or plastic). This is illustrated more clearly in the close up view of Figure 3.

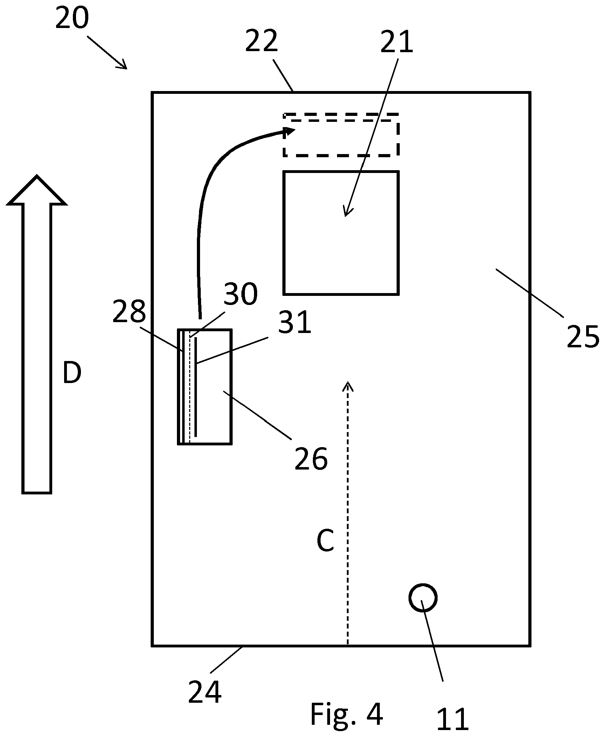

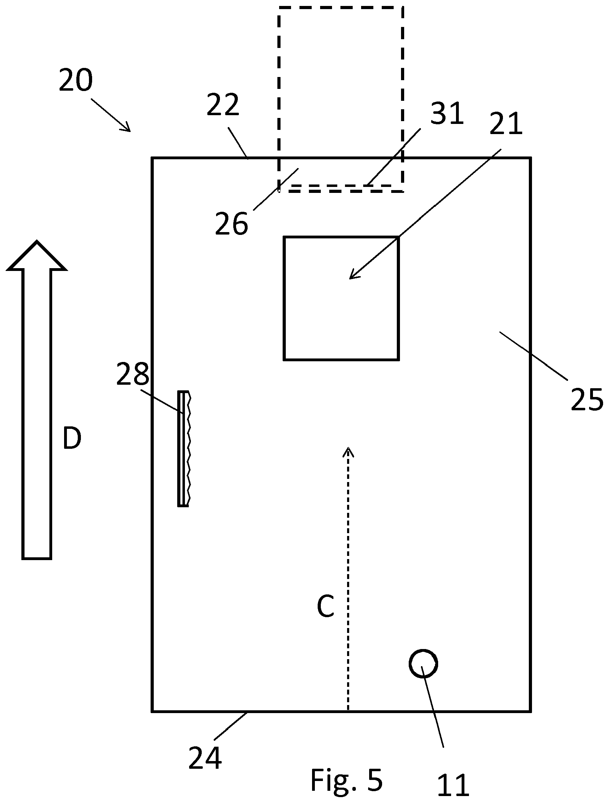

In use, the blanket 20 is laid longitudinally over the patient, the top end 22 of the blanket 20 being located at the neck of the patient, the bottom end 24 of the blanket 20 being located at the feet of the patient and the cut-out 21 being provided over the chest of the patient. A medical practitioner fully detaches the drape 26 from the blanket 20 (such that there is no longer a physical connection between the drape 26 and the blanket 20) by tearing along the breakable, perforated connection 30 (leaving a strip of material and the blanket connection adhesive on the blanket 20) and relocates and re-orients the drape 26 to its optimal use position and orientation 32 shown in dotted lines in Figure 4, namely at a location between the cut-out 21 and the top end 22 of the blanket 20 and rotated clockwise by 90° (relative to the blanket 20, typically in a plane of a planar surface of the drape 26). The medical practitioner removes the release liner covering the relocation adhesive and adheres the drape 26 to the blanket 20 at its new location and orientation 32. In the new position and orientation 32 of the drape 26, the line 31 of relocation adhesive is perpendicular to the longitudinal axis C (and to the line 28 of blanket connection adhesive which remains in its original position and orientation). As illustrated in Figure 5, in the new position and orientation 32, the drape 26 can then be pulled over the head of the patient to thereby shield the face of the patient from blood spatter and to inhibit the loss of air heated by the forced air warming blanket 20 in use. The forced air warming blanket 20 can then be used together with the drape 26 to regulate the temperature of the patient in a medical or surgical procedure or operation, typically to prevent unintended hypothermia (e.g. when the patient is under anaesthetic).

The drape 26 may be folded or unfolded when attached to the blanket 20 during manufacture. If it is folded, in use the drape may be unfolded after it has been relocated and re-oriented to new location and orientation 32 (as shown in Figure 5) to thereby cover the face of the patient.

It will be understood that, instead of adhering the detached drape 26 to the blanket after it has been detached from the blanket 20, the drape 26 could be adhered to the patient or to a garment worn by the patient. Alternatively, the detached drape 26 may be draped over the patient without being adhered to the blanket 20 or to the patient. In this case, the relocation adhesive arranged along line 31 is not required. It will also be understood that the perforated connection 30 could be replaced by any suitable detachable connection. For example, the drape 26 may alternatively be attached to the blanket 20 by way of a relatively weak adhesive connection (e.g. provided by a polymer adhesive layer), such that the drape 26 may be peeled off the blanket 20, or by a (e.g. plastic) zipper connection (such as a Ziplock® connection or similar) such that unzipping the zipper connection releases the drape 26 from the blanket 20.

Further variations and modifications may be made within the scope of the invention herein described. For example, markings or lead-ins may be provided on the layer of the blanket to which the drape is attached to identify its location. This is particularly useful given when the drape is transparent (as is typical).

Although in the examples described above, the line 8, 28 along which the blanket connection adhesive is provided is parallel to the principal manufacturing direction D, it may be that the line 8 along which the blanket connection adhesive is provided is substantially parallel to the direction D. For example, it may be that the nozzle which deposits the adhesive is imperfect such that it does not always eject adhesive onto the air impermeable layer 5, 25 in precisely the same direction, which may lead to the line 8, 28 deviating from the direction D, typically by an angle of less than 10° (preferably less than 5°) to the direction D.

Rather than being provided on the air impermeable layer 5, the blanket connection adhesive (and the drape) may instead be provided on the air permeable layer (in which case the air permeable layer is typically deposited on the air impermeable layer before heat sealing during manufacture rather than the other way round).

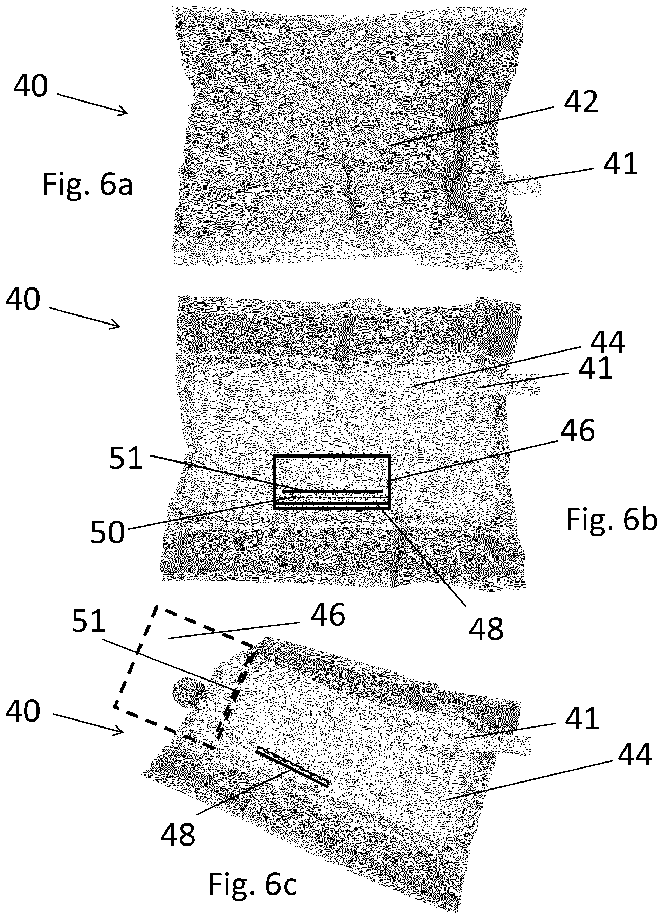

In addition, the detachable, relocatable drape 26 is not limited for use on the blankets illustrated in Figures 1 to 5 and it will be understood that a detachable, relocatable drape 26 may be provided on any shape of blanket. In one example, Figures 6a-6b show a rectangular full body forced air warming blanket 40 having an air permeable side 42 and an air impermeable side 44 opposite the air permeable side 42 with an air chamber there between. An air inlet port 41 is provided on the air impermeable side 44 which is configured to receive temperature conditioned air by way of a hose. Figure 6c shows the blanket 40 in use to regulate the temperature of a child patient, the air permeable side 42 facing the patient and the air impermeable side 44 facing away from the patient. In this case, a detachable, relocatable drape 46 is provided on (for example) the air impermeable side 44 of the forced air warming blanket 40 as shown schematically in Figure 6b. The drape 46 is adhered to the air impermeable side 44 of the forced air warming blanket 40 by blanket connection adhesive 48 arranged along a line parallel to the manufacturing direction during manufacture of the blanket 40 in the way described above with respect to Figure 2, and detached (for example) by tearing through perforations 50 (provided between the drape 46 and the blanket connection adhesive 48) and relocated and re-oriented in use (see Figure 6c), before being adhered to the blanket 40 (or alternatively the patient) in its new location and orientation by way of relocation adhesive 51 as set out above with respect to Figures 2 to 5. In the example of Figure 6c, the drape 46 covers the head of the patient to inhibit heat loss from gaps between the head of the patient and the blanket and to shield the face of the patient from blood spatter. Although Figure 6c shows the blanket 40 on top of the patient, it may alternatively by provided under the patient (again with the air permeable layer facing the patient and the air impermeable layer facing away from the patient). In this case, the drape 46 primarily shields the face of the patient from blood spatter in use.

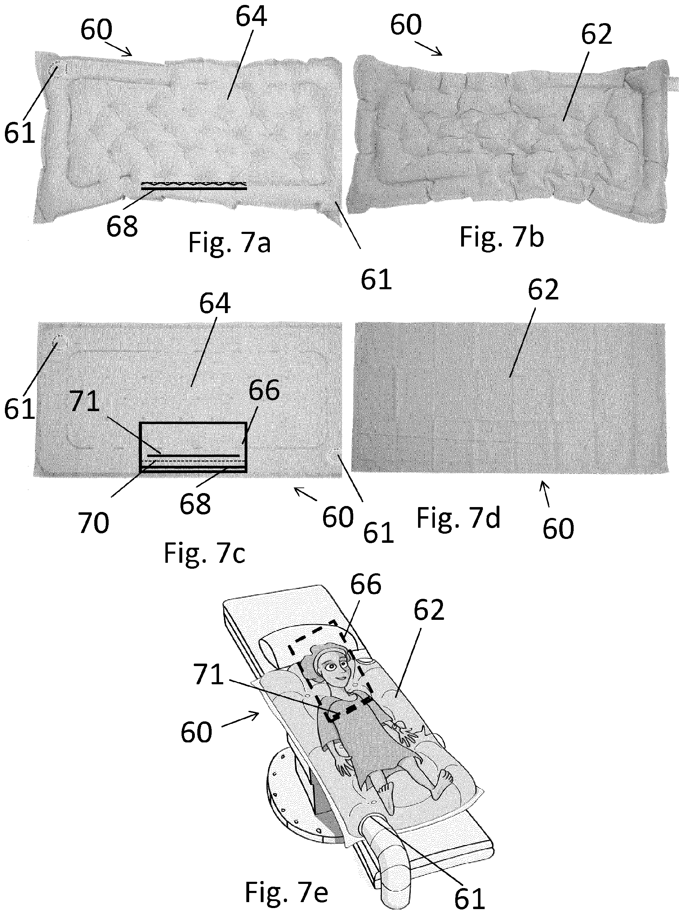

In another example, Figures 7a-7e show another full body rectangular forced air warming blanket 60 having an air permeable side 62 and an air impermeable side 64 opposite the air permeable side 62 with an air chamber there between. Air inlet ports 61 are provided at opposing ends of the air impermeable side 64 which are each configured to receive temperature conditioned air by way of a hose. Figure 7e shows the blanket 60 in use to regulate the temperature of an adult patient, the air permeable side 62 facing the patient and the air impermeable side 64 facing away from the patient. The blanket 60 is provided beneath the patient. In this case, a detachable, relocatable drape 66 is provided on (for example) the air impermeable side 64 of the forced air warming blanket 60 as shown schematically in Figure 7c. The drape 66 is adhered to the air impermeable side 64 of the forced air warming blanket 60 by a line of blanket connection adhesive 68 arranged along a line parallel to the manufacturing direction during manufacture of the blanket 60 in the way described above with respect to Figure 2, and detached (for example) by tearing through perforations 70 (provided between the drape 66 and the blanket connection adhesive 68) and relocated and re-oriented in use, before being adhered to the chest of the patient (over the patient's gown) in its new location and orientation by way of relocation adhesive 71 (see Figure 7e). In the example of Figure 7e, the drape 66 covers the head of the patient primarily to shield the face of the patient from blood spatter. Figure 7a shows the blanket 60 with the drape 66 having been detached.

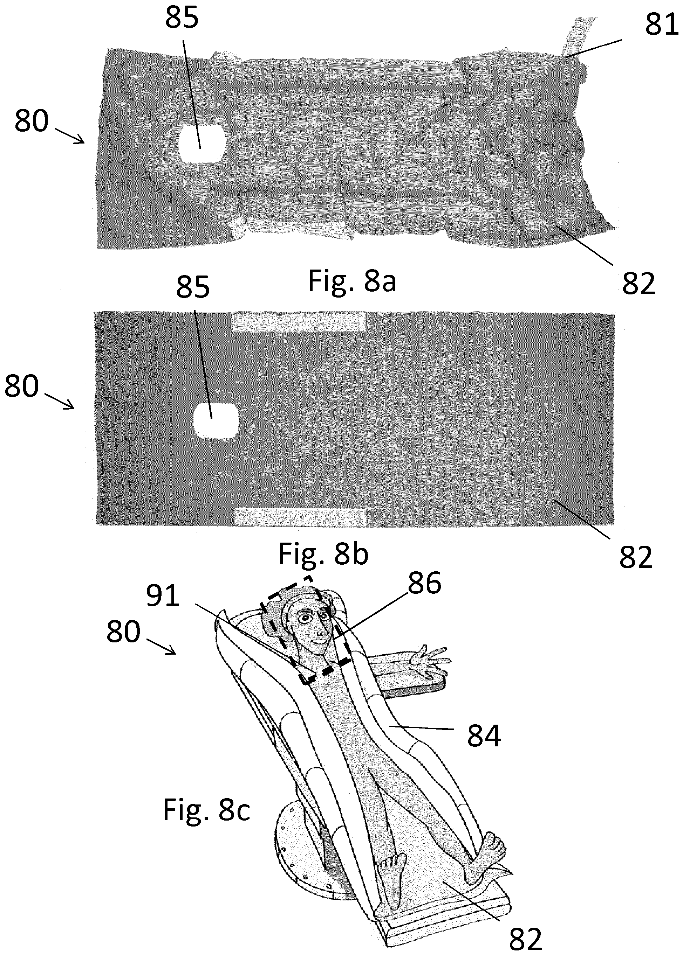

In another example, Figures 8a-8c show another full body rectangular forced air warming blanket 80 having an air permeable side 82 and an air impermeable side 84 opposite the air permeable side 82 with an air chamber there between. A cut-out 85 is provided 84 closer to one end of the blanket than to the other centrally with respect to the width of the blanket and extending through both the air permeable and impermeable sides 82. Cut-out 85 is a marker for the position of a patient's head. The head position marker leads to the patient being positioned in the right place more consistently. An air inlet port 81 is provided at an end of the air impermeable side 84 which is configured to receive temperature conditioned air by way of a hose. Figure 8c shows the blanket 80 in use to regulate the temperature of an adult patient, the air permeable side 82 facing the patient and the air impermeable side 84 facing away from the patient. The blanket 80 is provided beneath the patient but partially wrapped around the front of the patient. The blanket 80 covers the entire rear side of the body of the patient and part of the front side of the body of the patient. A detachable, relocatable drape 86 is adhered to the air permeable side 82 of the forced air warming blanket 80 by a line of blanket connection adhesive arranged along a line parallel to the manufacturing direction during manufacture of the blanket 80 in the way described above with respect to Figure 2, and detached (for example) by tearing through perforations provided between the blanket connection adhesive and the drape 86. The drape 86 is then relocated and re-oriented in use, before being adhered to the chest of the patient in its new location and orientation by way of relocation adhesive 91 (see Figure 8c). In the example of Figure 8c, the drape 86 covers the head of the patient primarily to shield the face of the patient from blood spatter.

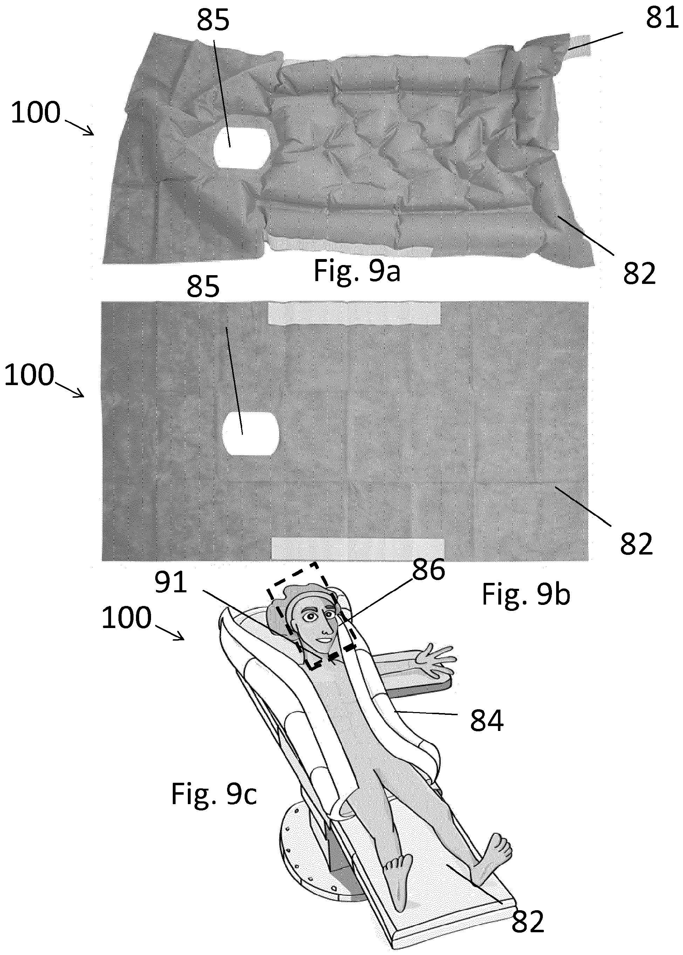

Figures 9a-9c show a similar forced air warming blanket 100 to the forced air warming blanket 80 shown in Figures 8a-8c, but the forced air warming blanket 100 is shorter than the forced air warming blanket 80 and covers only the rear of the upper body of the patient and a portion of the front of the upper body of the patient. Similar features between the embodiments of Figures 8a-c and Figures 9a-c are labelled with the same reference numerals used in Figures 8a-c.

It will be understood that, during manufacture, by orienting the constituent layers of the blanket such that their longitudinal axes are at a non-zero degrees angle to the manufacturing direction, the blanket connection adhesive may be arranged along a line extending in a direction which is at a non-zero degrees angle (which can typically be up to around 45°) to a direction parallel to the longitudinal axes of the constituent layers of the forced air warming blanket (and thus typically at a non-zero degrees angle to the longitudinal axis of the forced air warming blanket). However, most typically the blanket connection adhesive is arranged along a line parallel or substantially parallel to the longitudinal axis of the forced air warming blanket.

## Claims

1. A forced air warming blanket for regulating a temperature of a patient, the blanket having a patient drape detachably connected thereto.

2. The forced air warming blanket according to claim 1 wherein the patient drape is attached thereto by way of a blanket connection adhesive.

3. The forced air warming blanket according to claim 2 wherein the forced air warming blanket is elongate along a longitudinal axis, and wherein the said blanket connection adhesive is arranged along a line parallel or substantially parallel to the said longitudinal axis.

4. The forced air warming blanket according to any one preceding claim wherein the patient drape comprises a drape relocation adhesive provided thereon and covered by a release liner, the said release liner being removable to thereby allow the drape to be attached to the blanket or to a patient or to a garment worn by the patient by way of the drape relocation adhesive.

5. The forced air warming blanket according to any one preceding claim wherein the patient drape is detachable therefrom by hand.

6. The forced air warming blanket according to any one preceding claim wherein the patient drape is detachably connected thereto by way of a breakable connection.

7. The forced air warming blanket according to claim 6 wherein the said breakable connection comprises one or more perforations.

8. A method of manufacturing a forced air warming blanket for regulating a temperature of a patient, the method comprising: translating one or more constituent layers of the forced air warming blanket along a principal manufacturing direction; depositing blanket connection adhesive on a said constituent layer of the forced air warming blanket along a line parallel or substantially parallel to the principal manufacturing direction; and attaching a detachable patient drape to the forced air warming blanket by way of the blanket connection adhesive.

9. A method of using a forced air warming blanket for regulating a temperature of a patient, the blanket having a patient drape detachably connected thereto, the method comprising: detaching the patient drape from the forced air warming blanket; and relocating and/or re-orienting the patient drape.

10. The method according to claim 9 further comprising regulating a temperature of a patient by way of the forced air warming blanket after the steps of detaching and relocating and/or re-orienting the patient drape.

11. The method according to claim 9 or claim 10 comprising relocating and/or re-orienting the patient drape to thereby cover a portion of a patient.

12. The method according to any one of claims 9 to 11 wherein the step of relocating and/or re-orienting the patient drape comprises adhering the patient drape to the blanket or to a or the patient or to a garment worn by the patient.

13. The method according to claim 12 wherein the step of adhering the patient drape to the blanket or to a or the patient comprises removing a release liner from a drape relocation adhesive provided on the drape and adhering the patient drape to the blanket or to the patient or to a garment worn by the patient by way of the drape relocation adhesive.

14. The method according to any one of claims 9 to 13 wherein the step of relocating and/or re-orienting the patient drape comprises re-orienting the patient drape relative to the forced air warming blanket.

15. The method according to any one of claims 9 to 14 wherein the step of detaching the patient drape from the forced air warming blanket comprises detaching the patient drape from the forced air warming blanket by hand.
